# EUROPÄISCHE PATENTSCHRIFT

(11) **EP 1 498 688 B1**
(45) Veröffentlichungstag und Bekanntmachung des Hinweises auf die Patenterteilung: **21.09.2005**
(21) Anmeldenummer: 03016240.8
(22) Anmeldetag: 17.07.2003
(51) Int. Cl.: G01B 11/00, G01B 11/24, G01B 11/245, G01B 21/04

(54) **Lokator und optisches Messsystem**
Probe and optical measuring system
Sonde et dispositif optique de mesure

(43) Veröffentlichungstag der Anmeldung: 19.01.2005
(73) Patentinhaber: AXIOS 3D Services GmbH, 26121 Oldenburg (DE)
(72) Erfinder: Broers, Holger, 26670 Uplengen-Spols (DE)
(74) Vertreter: Heinze, Ekkehard

(56) Entgegenhaltungen:
- WO-A-02/01148
- US-A- 6 166 809
- US-B1- 6 389 158

## Beschreibung

Die Erfindung betrifft einen Lokator eines sogenannten optischen Navigationssystems zur Positions- bzw. Lagebestimmung von Körpern im Raum sowie ein Navigationssystem, das mit derartigen Lokatoren arbeitet.

Optische Messsysteme dieser Art, mit denen Punkte und Lagebeziehungen auf bzw. zwischen Körpern im Raum vermessen werden können, sind bekannt und von verschiedenen Herstellern auf dem Markt. Vereinfacht gesagt, bestehen diese Messsysteme aus einem optischen Aufbau mit Lokatoren und einer Kameraanordnung, einem Laserscanner o.ä. sowie einer Datenverarbeitungs- bzw. Rechnerkomponente, die an die optische Komponente angeschlossen und mit einer geeigneten Software zur Auswertung einer Mehrzahl aufeinanderfolgend aufgenommener Bilder ausgestattet ist. Derartige Systeme finden Anwendung insbesondere in der Roboter- und Handhabungstechnik sowie - in neuerer Zeit - auch in der Medizin. Dort unterstützt ein solches System beispielsweise einen Operateur durch Bereitstellung präziser Orts- bzw. Lageinformationen für einen Operationsbereich, etwa bei einem minimal-invasiven Eingriff oder der Implantation einer Gelenkprothese.

Ein typisches optisches System dieser Art umfasst zwei auf einer festen Basis konvergent angebrachte Kameras, deren relative und innere Orientierung vorab im Labor bestimmt werden und die durch einen geeignet stabilen mechanischen Aufbau in einer absolut invariablen Lage- und Blickfeldbeziehung zueinander stehen.

Im Blickfeld der Kameraanordnung liegende aktive oder passive Signalgeber dienen zur Markierung von Objektpunkten, deren räumliche Lage zu bestimmen ist. Unter passiver Signalisierung wird dabei eine Markierung mit sogenannten retro-reflektierenden Reflektorelementen (insbesondere Kugeln) im Zusammenwirken mit einer aus der Aufnahmerichtung strahlenden Beleuchtung verstanden. Derartige Signalgeber liefern stark reflektierende punktartige Signale und ermöglichen die Bestimmung von Näherungswerten der Bildpunkte mittels einfacher Schwellwertverfahren. Bei der aktiven Signalisierung umfasst der Lokator Leuchtdioden, die über ein Steuergerät angesteuert werden. Mit Hilfe der elektronischen Steuerung einzelner Signalisierungen ist eine Kodierung und damit eindeutige Kennzeichnung der einzelnen Signalgeber möglich. Diese resultiert in einer höheren Zuverlässigkeit, die hierzu benötigte Stromversorgung bedingt aber einen erhöhten Installations- und Wartungsaufwand, und die üblicherweise vorhandenen Anschlussleitungen erschweren die Handhabung des Systems und verringern dessen Flexibilität im Einsatz.

Üblicherweise werden mehrere Signalgeber unter Herstellung einer starren Lagebeziehung zueinander mittels eines Grundkörpers zu einem (passiven oder aktiven) Lokator zusammengefasst. Üblicherweise weisen derartige Lokatoren drei oder vier Signalgeber - z.B. retro-reflektierende Kugeln - auf, was anhand geeigneter, dem Fachmann bekannter und daher hier nicht weiter zu erläuternder Algorithmen eine eindeutige Positionsbestimmung eines mit dem Lokator markierten Objektpunktes im Raum ermöglicht. Falls ein vierter Signalgeber vorgesehen ist, ist dieser im Grunde redundant, ermöglicht aber die Zuverlässigkeit der Messung erhöhende Prüfungen.

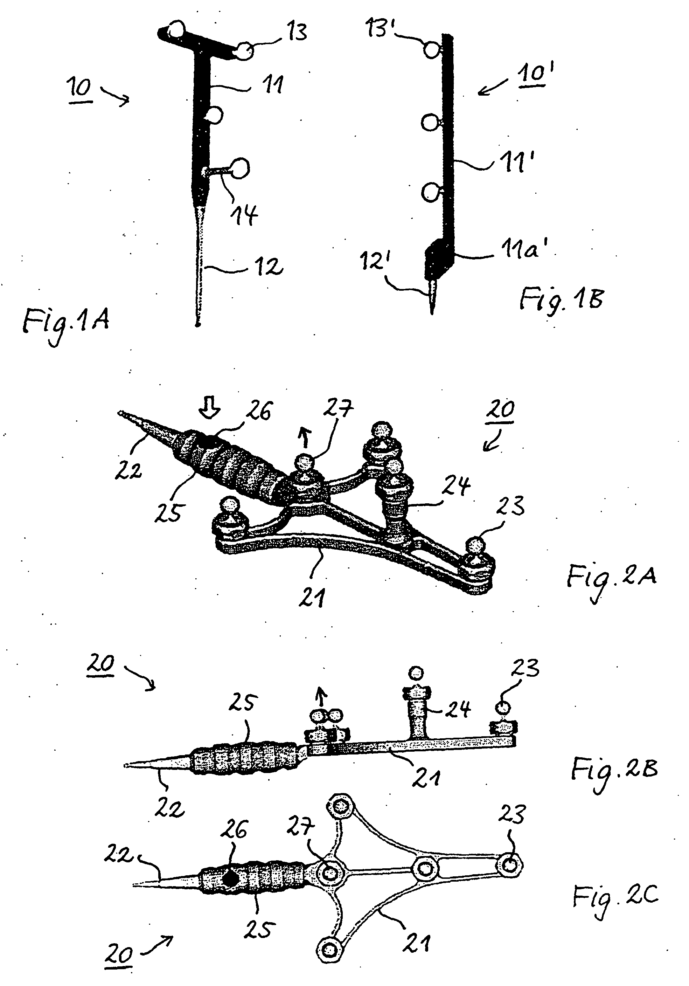

Eine vorgegebene Lokatorgeometrie wird bei passiven Lokatoren in eine Rechnereinheit geladen. Bei aktiven Lokatoren können die lokalen Punktinformationen auf einem im Lokator eingebauten nicht-flüchtigen Speicher (z.B. S-ROM) gespeichert werden. Im Programmspeicher der Datenverarbeitungskomponente (Steuer- und Auswertungseinheit) stehen Algorithmen bereit, mit denen aufgrund gemessener "globaler" Koordinaten und der eingelernten "lokalen" Koordinaten bzw. lokatorbezogenen Koordinaten die gewünschte Positions- bzw. Lagebestimmung des zu vermessenden Körpers im Raum gelingt. Beispiele bekannter passiver Lokatoraufbauten sind in Fig. 1A und 1B gezeigt und werden weiter unten beschrieben.

Bekannt und im praktischen Einsatz sind passive wie aktive Lokatoren eines ersten Typs, der Befestigungsmittel zur starren Anbringung an einem zu vermessenden Körper (beispielsweise an einer Extremität eines Menschen für den Einsatz im medizinischen Bereich) aufweist, sowie sogenannte Handtaster mit einer Antastspitze, die an Referenzpunkten im lokalen Koordinatensystem kalibriert ist. Die Lage der Antastspitze und damit eines von dieser berührten Punkten eines zu vermessenden Körpers im Raum wird über die Kamera-Erfassung der Signale der Lokator-Signalgeber und unter Einsatz eines Transformationsalgorithmus zur Transformation in den Objektraum ermittelt.

Die Vermessung von Körpern oder gar von Lagebeziehungen zwischen Körpern im Raum erfordert eine Anzahl von Mess- und Auswertungsschritten, die jeweils nach Positionierung der Antastspitze an einem Oberflächenpunkt des Körpers durchzuführen sind. Jeder Schritt ist hierbei durch den Vermesser auszulösen. Hierfür sind verschiedene Lösungen bekannt, etwa die Übermittlung eines Fernsteuersignals über eine Infrarotverbindung zwischen dem Vermesser und der Steuer- und Auswertungseinheit oder die leitungsgebundene Übermittlung eines mit einem Fußschalter erzeugten Auslösesignals oder die Ausführung einer vorgeschriebenen Bewegung des Lokators, die von der Kameraanordnung erfasst und aufgrund eines geeigneten Auswertungsalgorithmus im Sinne eines Auslösesignals interpretiert wird.

WO-A-02/01148 offenbart einen Lokator eines optischen Messsystems zur Positions- und/oder Lagebestimmung von Körpern im Raum, wobei der Lokator mehrere Reflektorelemente an einem starren Grundkörper aufweist. Dabei weist das Messsystem eine Aufnahmeeinrichtung zur Aufnahme von durch die Reflektorelemente an Lokatoren erzeugten oder reflektierten Lichtsignale und eine mit der Aufnahme Einrichtung verbundene Steuer- und Auswertungseinrichtung zur Steuerung und Ausführung der Positionsbestimmung aufgrund der in mehreren Erfassungsschritten gewonnenen Aufnahmen der Aufnahmeeinrichtung auf. Der Lokator besitzt weiterhin einen mechano-optischen Schalter zum Auslösen der Erfassungsschritte der Aufnahmeeinrichtung und/oder von Auswertungsschritten in der Steuer- und Auswertungseinrichtung, wobei der mechano-optischen Schalter die Größe, die Schattierung oder die Farbe des Reflektorelements ändert und dadurch das Auslösen bewirkt.

Diese bekannten Verfahren erfordern entweder einen relativ hohen Hardware- und Installationsaufwand oder gestalten sich umständlich für den Vermesser, oder es treten beide (und weitere) Nachteile kombiniert auf.

Der Erfindung liegt daher die Aufgabe zugrunde, einen verbesserten Lokator der gattungsgemäßen Art bereitzustellen, der die Auslösung von Mess- bzw. Auswertungsschritten in einem optischen Navigationssystem auf besonders einfache und zuverlässige Weise ermöglicht. Weiterhin liegt der Erfindung die Aufgabe der Bereitstellung eines entsprechenden Navigationssystems zugrunde.

Diese Aufgabe wird in ihrem ersten Aspekt durch einen Lokator mit den Merkmalen des Anspruchs 1 und in ihrem zweiten Aspekt durch ein optisches Messsystem mit den Merkmalen des Anspruchs 6 gelöst.

Die Erfindung schließt den wesentlichen Gedanken ein, ein zusätzliches, eine eigene Energieversorgung erforderndes Signalisierungssystem zum Auslösen der Mess- bzw. Auswertungsschritte dadurch zu vermeiden, dass das optische Navigationssystem selbst die Auslöse-Funktionalität realisiert. Sie schließt weiter den Gedanken ein, dem Nutzer nur einen minimalen Betätigungsaufwand zuzumuten. Schließlich gehört zur Erfindung der hieraus abgeleitete Gedanke, im Lokator selbst einen mechano-optischen Schalter zum Auslösen der Erfassungs- bzw. Auswertungsschritte vorzusehen, so dass der Benutzer im Rahmen der normalen Handhabung des Lokators die Messungen per Tastendruck initiieren kann.

Eine bevorzugte Ausführung der Erfindung zeichnet sich dadurch aus, dass der mechano-optische Schalter ein bewegbares, gegenüber der Menge der zur Positionsbestimmung benutzten Reflektorelemente überzähliges Reflektorelement aufweist. In einer speziellen Fortbildung dieser Ausführungsform, die aus derzeitiger Sicht besonders für die Praxis geeignet ist, weist der mechano-optische Schalter einen federnd rückstellenden Tastschalter auf. Hierbei ist insbesondere der Tastschalter über einen federbelasteten Schwenkhebel mit dem bewegbaren überzähligen Reflektorelement verbunden. Dies stellt einen besonders einfachen und robusten mechanischen Aufbau dar, mit dessen Ausführung sich die Gestehungskosten des Lokators nicht dramatisch erhöhen.

Sofern der erfindungsgemäß ausgerüstete Lokator eine Handhabe (beispielsweise einen geriffelten Kunststoff- oder Elastomer-Handgriff) aufweist, an dem der Benutzer ihn ergreift, so ist der Tastschalter bevorzugt in dieser Handhabe angeordnet, und zwar in besonders vorteilhafter Weise in einer typischen "Daumen- oder Fingerposition". Dies ist natürlich besonders vorteilhaft bei einer Ausführung als handgeführter Taster mit einer, insbesondere im wesentlichen kugelförmigen, Tasterspitze.

Alternativ zur Ausführung als handgeführter Taster ("Handtaster") kann der Lokator auch als körperfester Lokator mit den erfindungsgemäßen Merkmalen ausgestattet sein und in dieser Ausführung Befestigungsmittel zur starren Befestigung an dem der Positionsbestimmung zu unterziehenden Körper haben, die insbesondere zur starren Anbringung an einer Extremität eines Säugetiers ausgebildet sind. Wenn beispielsweise ein solcher Lokator als knochenfester Lokator an einer Extremität eines Menschen angebracht wird, um Basisdaten zu deren Lage und Orientierung im Raum zu liefern, werden auch hier üblicherweise mehrere Messschritte unter zwischenzeitlicher Bewegung der Extremität ausgeführt. Der Arzt kann dann, wenn er den Lokator erfasst und mit ihm die Extremität bewegt, ohne zusätzliche Handhabungen die Messschritte direkt am Lokator auslösen.

Vorteile und Zweckmäßigkeiten der Erfindung ergeben sich im übrigen aus den abhängigen Ansprüchen sowie der nachfolgenden Beschreibung eines bevorzugten Ausführungsbeispiels anhand der Figuren. Von diesen zeigen:
- Fig. 1A und 1B: Skizzen herkömmlicher Lokatoren (handgeführter Taster),
- Fig. 2A bis 2C: eine perspektivische Ansicht sowie Seitenansicht und Draufsicht eines handgeführten Tasters gemäß einer Ausführungsform der Erfindung,
- Fig. 3: eine Längsschnittdarstellung des handgeführten Tasters nach Fig. 2A bis 2C und
- Fig. 4: ein Ablaufdiagramm zur Verdeutlichung des Betriebs eines optischen Navigationssystems unter Einsatz eines erfindungsgemäßen Lokators.

Fig. 1A und 1B zeigen zwei unterschiedliche handgeführte Taster 10 bzw. 10' nach dem Stand der Technik. Der Handtaster 10 nach Fig. 1A hat einen T-förmigen Kunststoff-Grundkörper 11 und eine an das untere Ende des "T" angesetzte, langgestreckte Antastspitze 12. An den Enden des Querbalkens des "T" ist je eine retro-reflektierende Kugel 13 als Reflektorelement angesetzt, und zwei weitere retro-reflektierende Kugeln 13 befinden sich etwa auf halber Strecke zwischen dem Querbalken und dem proximalen Ende der Antastspitze sowie in der Nähe dieses proximalen Endes. Die letztere retro-reflektierende Kugel sitzt auf einem Stift 14, der einen vorbestimmten Abstand zum Grundkörper 11 schafft.

Der handgeführte Taster 10' nach Fig. 1B hat einen im wesentlichen linearen Grundkörper 11', der an einem Ende eine Verdickung 11a' aufweist, in die eine kurze Antastspitze 12' eingesetzt ist. Im geraden Abschnitt des Grundkörpers 11' sind in gleichen Abständen drei retro-reflektierende Kugeln 13' in gleicher Höhenlage bezüglich des Grundkörpers angeordnet.

Fig. 2A bis 2C zeigen einen erfindungsgemäßen handgeführten Taster 20, dessen Grundaufbau im Hinblick auf das Vorhandensein eines Kunststoff-Grundkörpers 21 und einer metallenen Antastspitze 22 dem Aufbau der bekannten Handtaster ähnelt. Des weiteren sind auch hier mehrere retro-reflektierende Kugeln 13 als Reflektorelemente bzw. Signalgeber des Lokators vorgesehen, und eine hiervon ist (ähnlich wie bei dem Handtaster 10 nach Fig. 1A) durch einen Pfosten bzw. Ständer 24 gegenüber der Erstreckungsebene des Grundkörpers 21 erhöht angeordnet.

Abweichend von den bekannten Handtastern gemäß Fig. 1A und 1B, hat der erfindungsgemäße Handtaster 20 jedoch einen mehrfach verstrebten, in der Draufsicht annähernd dreieckigen Grundkörper. Weiterhin hat er einen balligen und geriffelten Handgriff 25, in dem ein Tastschalter 26 (nahe dem der Antastspitze 22 benachbarten Ende des Handgriffes) angeordnet ist. Nahe dem anderen Ende des Handgriffes 25 ist im Grundkörper 21 eine zusätzliche (fünfte) retro-reflektierende Kugel 27 vorgesehen, die über einen (in den Außenansichten nicht zu erkennenden) federnd gelagerten Schwenkbügel mit dem Tastschalter 26 verbunden ist.

Wird der Tastschalter mit dem Daumen oder einem Finger des Benutzers niedergedrückt, wie durch den dicken Pfeil in Fig. 2A symbolisiert, so führt die zusätzliche retro-reflektierende Kugel 27 eine Schwenkbewegung nach oben aus, wie durch den einfach gezeichneten Pfeil in den Figuren 2A und 2B symbolisiert.

Diese Kugelbewegung stellt eine von jedweden übrigen Bewegungen des Handtasters, bei denen die von den übrigen retro-reflektierenden Kugeln 23 gelieferten Signale eine gewisse Synchronität aufweisen, durch eine der Kameraanordnung nachgeschaltete Auswertungseinrichtung unterscheidbare Signalgabe dar. Wird von der Steuer- und Auswertungseinrichtung des Navigationssystems ein solches isoliertes Signal empfangen, wird dies als Auslösesignal für einen Mess- bzw. Auswertungsschritt gewertet. Es kann sich hierbei einerseits um die Auslösung eines neuen Messvorganges in einer neuen Tasterposition handeln, andererseits aber auch um einen wiederholten Auswertungsschritt, falls in einer bestimmten Tasterposition keine geltenden Koordinaten gefunden werden konnten.

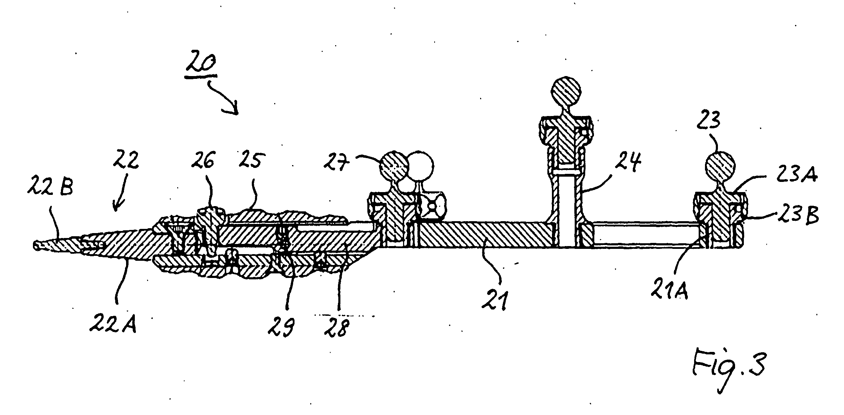

Fig. 3 zeigt den Aufbau des handgeführten Tasters 20 in einer Längsschnittdarstellung etwas genauer. Es ist zu erkennen, dass die retro-reflektierenden Kugeln 23 jeweils auf einem zapfenartigen Grundkörper 23A angeordnet sind, welcher wiederum in einem zentrisch durchbohrten Halter 23B ruht, über den schließlich die Fixierung in angepassten Durchgangsbohrungen 21A des Grundkörpers 21 erfolgt. Wie bereits erwähnt, erfolgt die Anbringung der erhöht angeordneten retro-reflektierenden Kugel in der Mitte der Reflektoranordnung über einen Pfosten 24, der an einem Ende den entsprechenden Halter 23B aufnimmt und am anderen Ende in der zugehörigen Bohrung 21A fixiert ist. Weiterhin ist zu erkennen, dass die Antastspitze 22 ein proximales Teil 22A und ein in dieses am verjüngten Ende eingesetztes distales Teil 22B umfasst.

Besonders hinzuweisen ist auf den Bereich der Handhabe 25, in deren Innerem ein sich zwischen dem Tastschalter 26 und der bewegbaren retro-reflektierenden Kugel 27 erstreckender Schwenkhebel 28 zu erkennen ist, der um eine senkrecht zur Längserstreckung des Handgriffs verlaufende Schwenkachse 29 schwenkbar ist. Nahe dem einen Ende dieses Schwenkhebels 28 ist der Tastschalter 26 in eine entsprechende (nicht gesondert bezeichnete) Bohrung eingepasst, und am anderen Ende ist die retro-reflektierende Kugel 27 mit dem weiter oben bereits erwähnten "Unterbau" aus Grundkörper 23A und Halter 23B in einer weiteren Bohrung des Schwenkhebels fixiert. Unterhalb des Tastschalters 26 ist eine (in der Zeichnung nicht dargestellte) Druckfeder angeordnet, die für eine Federvorspannung des Schwenkhebels 28 in die in der Figur gezeigte Ausgangsstellung und eine Rückführung in diese nach einer Betätigung sorgt.

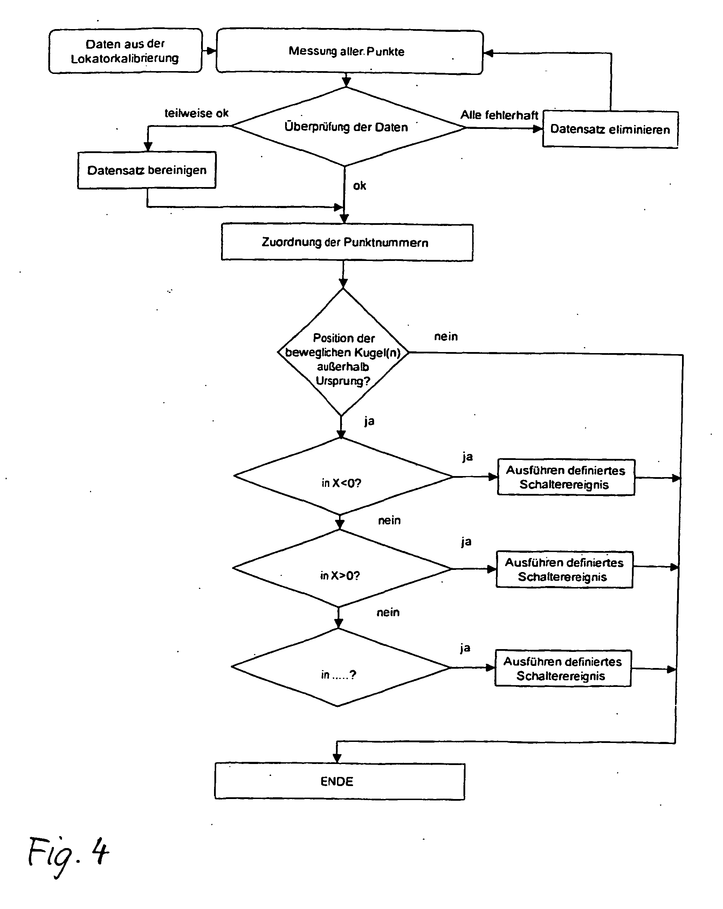

Fig. 4 zeigt ein Ablaufdiagramm, welches den Einsatz eines erfindungsgemäßen Lokators in einem optischen Navigationssystem erläutert und das im wesentlichen selbsterklärend ist.

Die Erfindung ist nicht auf das hier dargestellte Beispiel beschränkt, sondern eben so in einer Vielzahl von Abwandlungen ausführbar, die im Rahmen fachgemäßen Handelns liegen. Insbesondere bestehen Abwandlungsmöglichkeiten in der Form und dem Material des Grundkörpers und des Handgriffs, wobei letzterer auch fortgelassen sein kann. Es sei auch besonders darauf hingewiesen, dass die Erfindung auch mit andersartigen Reflektorelementen und auch bei einem Lokator vom aktiven Typ anwendbar ist.

## Patentansprüche

1. Lokator (20) eines optischen Messsystems zur Positions- und/oder Lagebestimmung von Körpern im Raum, wobei der Lokator mehrere Reflektorelemente (23, 27) an einem starren Grundkörper (21) aufweist und das Messsystem ein Aufnahmeeinrichtung zur Aufnahme von durch die Reflektorelemente an Lokatoren erzeugten oder reflektierten Lichtsignale und eine mit der Aufnahmeeinrichtung verbundene Steuer- und Auswertungseinrichtung zur Steuerung und Ausführung der Positionsbestimmung aufgrund der in mehreren Erfassungsschritten gewonnenen Aufnahmen der Aufnahmeeinrichtung aufweist, wobei der Lokator weiterhin einen mechano-optischen Schalter (26-29) zum Auslösen der Erfassungsschritte der Aufnahmeeinrichtung und/oder von Auswertungsschritten in der Steuer- und Auswertungseinrichtung aufweist,
**dadurch gekennzeichnet, dass**
der mechano-optische Schalter (26-29) ein bewegbares, gegenüber der Menge der zur Positionsbestimmung benutzten Reflektorelemente (23) überzähliges Reflektorelement (27) aufweist und zu dessen Bewegung ausgebildet ist, welche ihrerseits das Auslösen bewirkt.

2. Lokator nach Anspruch 1,
**dadurch gekennzeichnet, dass**
der mechano-optische Schalter (26-29) einen federnd rückstellenden Tastschalter (26) aufweist.

3. Lokator nach Anspruch 2,
**dadurch gekennzeichnet, dass**
der Tastschalter über einen federbelasteten Schwenkhebel (28) mit dem bewegbaren überzähligen Reflektorelement (27) verbunden ist.

4. Lokator nach einem der Ansprüche 1 bis 3,
**dadurch gekennzeichnet, dass**
die Relektorelemente (23, 27) als retro-reflektierende Kugeln ausgebildet sind.

5. Lokator nach einem der Ansprüche 1 bis 4,
**gekennzeichnet durch**
Befestigungsmittel zur starren Befestigung an dem der Positionsbestimmung zu unterziehenden Körper, die insbesondere zur starren Anbringung an einer Extremität eines Säugetiers ausgebildet sind.

6. Optisches Messsystem zur Positions- und/oder Lagebestimmung von Körpern im Raum, das eine Aufnahmeeinrichtung zur Aufnahme von durch Reflektorelemente an Lokatoren erzeugte oder reflektierte Lichtsignale und eine mit der Aufnahmeeinrichtung verbundene Steuer- und Auswertungseinrichtung zur Steuerung und Ausführung der Positionsbestimmung aufgrund der in mehreren Erfassungsschritten gewonnenen Aufnahmen der Aufnahmeeinrichtung, und mindestens einen Lokator nach einem der vorangehenden Ansprüche aufweist,
**dadurch gekennzeichnet, dass**
die Steuer- und Auswertungseinrichtung Auslösemittel zur Erfassung einer von einer gemeinsamen Zeitabhängigkeit mehrerer Lichtsignale eines Lokators unabhängigen Zeitabhängigkeit eines einzelnen Lichtsignals des Lokators in den Bildern der Aufnahmeeinrichtung und zum Auslösen der Erfassungsschritte und/oder Auswertungsschritte im Ansprechen hierauf aufweist.

## Claims

1. Locator (20) of an optical measurement system for determining the position and/or location of bodies in space, the locator comprising a plurality of reflector elements (23, 27) on a rigid base body (21), and the measurement system comprising a pick-up means for picking up light signals generated or reflected by the reflector elements at the locators, and a control and evaluation unit associated with the pick-up means for controlling and performing the positional determination based on pick-ups obtained by the pick-up means in several detection steps, the locator moreover comprising a mechanical-optical switch (26-29) for triggering the detection steps in the control and evaluation means,
**characterized in that**
the mechanical-optical switch (26-29) comprising a movable reflector element (27) that is redundant with respect to the number of reflector elements (23) used for the positional determination and formed for movement thereof, which in turn effects the triggering.

2. Locator according to claim 1,
**characterized in that**
the mechanical-optical switch (26-29) comprises a resiliently resetting push-button switch (26).

3. Locator according to claim 2,
**characterized in that**
the push-button switch is connected to the movable redundant reflector element (27) via a spring-loaded swivelling lever (28).

4. Locator according to claims 1 through 3,
**characterized in that**
the reflector elements (23, 27) are configured so as to be retro-reflecting spheres.

5. Locator according to claims 1 through 4,
**characterized by**
attachment means for rigidly attaching to the body to be subjected to the positional determination, which are in particular configured for being rigidly attached to a limb of a mammal.

6. Optical measurement system for determining the position and/or location of a body in space, comprising a pick-up means for picking up light signals generated or reflected by reflector elements at the locators, and a control and evaluation unit associated with the pick-up means for controlling and performing the positional determination based on pick-ups obtained by the pick-up means in several detection steps, and at least one locator according to any one of the preceding claims,
**characterized in that**
the control and evaluation unit comprises triggering means for detecting a time-dependence of a single light signal of the locator in the images of the pick-up means, which time-dependence is independent of a common time-dependence of several light signals of a locator, and for triggering the detection steps and/or evaluation steps in response thereupon.

## Revendications

1. Positionneur (20) d'un système de mesure optique pour déterminer la position et/ou le positionnement de corps dans l'espace, le positionneur présentant plusieurs éléments de réflecteur (23, 27) sur un corps de base (21) rigide, et le système de mesure présentant un dispositif de réception pour la réception de signaux lumineux générés ou réfléchis par les éléments de réflecteur sur des positionneurs et un dispositif de commande et d'exploitation relié au dispositif de réception pour commander et exécuter la détermination de position sur la base des réceptions du dispositif de réception obtenues en plusieurs étapes d'acquisition, le positionneur présentant en outre un commutateur mécano-optique (26 - 29) pour déclencher les étapes d'acquisition du dispositif de réception et/ou d'étapes d'exploitation dans le dispositif de commande et d'exploitation,
**caractérisé en ce que**
le commutateur mécano-optique (26 - 29) présente un élément de réflecteur (27) mobile, en surnombre par rapport à la quantité des éléments de réflecteur (23) utilisés pour la détermination de position, et est constitué pour son déplacement qui, de son côté, entraîne le déclenchement.

2. Positionneur selon la revendication 1,
**caractérisé en ce que**
le commutateur mécano-optique (26 - 29) présente un commutateur à touche (216) revenant élastiquement en arrière.

3. Positionneur selon la revendication 2,
**caractérisé en ce que**
le commutateur à touche est relié au travers d'un levier pivotant (28) chargé par ressort à l'élément de réflecteur (27) mobile en surnombre.

4. Positionneur selon l'une quelconque des revendications 1 à 3,
**caractérisé en ce que**
les éléments de réflecteur (23, 27) sont constitués en tant que boules rétro-réfléchissantes.

5. Positionneur selon l'une quelconque des revendications 1 à 4,
**caractérisé par**
des moyens de fixation pour une fixation rigide au corps à soumettre à la détermination de position, qui sont en particulier constitués pour le montage rigide en une extrémité d'un mammifère.

6. Système de mesure optique pour déterminer la position et/ou le positionnement de corps dans l'espace, présentant un dispositif de réception pour la réception de signaux lumineux générés ou réfléchis par les éléments de réflecteur sur des positionneurs, et un dispositif de commande et d'exploitation relié au dispositif de réception pour commander et exécuter la détermination de position sur la base des réceptions du dispositif de réception obtenues en plusieurs étapes d'acquisition, et au moins un positionneur selon l'une quelconque des revendications précédentes,
**caractérisé en ce que**
le dispositif de commande et d'exploitation présente des moyens de déclenchement pour l'acquisition d'une dépendance temporelle indépendante d'une dépendance temporelle commune de plusieurs signaux lumineux d'un positionneur d'un signal lumineux unique du positionneur dans les images du dispositif de réception et pour déclencher les étapes d'acquisition et/ou les étapes d'exploitation en réponse à cela.
